# EUROPEAN PATENT APPLICATION

(11) **EP 1 977 789 A1**
(43) Date of publication of application: **08.10.2008**
(21) Application number: 08005924.9
(22) Date of filing: 28.03.2008
(51) Int. Cl.: A61Q 17/04, A61K 8/81, A61K 8/06

(54) **Method for obtaining O/W cosmetic emulsions with high water resistance**

(30) Priority: 03.04.2007 IT MI20070689
(71) Applicant: 3V SIGMA S.p.A, 20121 Milano (IT)
(72) Inventor: Coccia, Maria Gabriella, 24100 Bergamo (IT)
(74) Representative: Minoja, Fabrizio

(57) **Abstract**

This invention relates to a method for obtaining cationic O/W emulsions designed for skin care, which present high water resistance associated with excellent "skin feeling" characteristics, consisting of incorporating a polymer obtainable by homopolymerisation of a monomer A or B or copolymerisation of A and B, wherein A and B are dialkylaminoalkyl acrylate or dialkylaminoalkyl methacrylate or their quaternary ammonium salts. The invention also concerns the compositions obtainable by the method.

## Description

### Field of application of the invention

This invention relates to a method for obtaining cationic O/W emulsions to be used for skin care, which present good viscosity and stability together with high water resistance, associated with excellent "skin feeling" characteristics and a low total polymer content. In particular, the method claimed involves the addition of a cationic polymer with formula I to an O/W emulsions free of polymers added specifically to increase the water resistance.

### Background

The water resistance and sensory properties of an O/W emulsion are characteristics very commonly required in skin care products. For example, water resistance is of crucial importance in sunscreen products, children's skin care products, insect-repellent compositions and make-up products.

Sensory properties are also important for any product designed to be applied to the skin, and particularly important for products which, for reasons of composition, tend to be somewhat sticky. Stickiness may be due to the presence of a high content of UV filters and/or film-forming polymers added specifically to increase the water resistance of the final composition.

Nowadays, the cosmetics industry is constantly searching for ingredients that perform more than one function in a composition.

This kind of result can be obtained by adding an effective quantity of homo- or copolymers as described below, as a single ingredient, according to the invention. The addition of said single ingredient produces different characteristics which, to date, have required the combined action of more than one ingredient: viscosity and stability, water resistance and excellent "skin feeling".

### Description of the invention

Surprisingly, it has been found that the addition of 0.1% to 10% by weight of homo- or copolymers as described below gives the O/W emulsions that contain them a high degree of water resistance together with excellent sensory properties, good viscosity and stability.

The water resistance of the cosmetic emulsions used for skin care is commonly obtained by adding a polymer or monomer additive that forms a protective water-repellent film on the treated skin. Examples include patent US 6274124, which claims increased water resistance of cosmetic formulations due to the addition of an appropriate quantity of 1,2-pentanediol, and many patents relating to the use of polymeric film formers, such as US 7060257, US 5725844 and US 5093107, wherein classic emulsions, viscosified with anionic polymers, are made resistant or highly resistant to water by the addition of 1-3% of vinylpyrrolidone copolymers, such as the copolymer PVP/Eicosene.

In all cases, the emulsions obtained are water resistant but characterised by a high content of polymers, which are not generally biodegradable, and/or a high degree of stickiness, especially in the case of cosmetic emulsions containing large amounts of organic sunscreens.

The subject of the invention is consequently a method of giving O/W emulsions high water resistance together with excellent sensory properties, good viscosity and stability, by incorporating in the emulsion 0.1 to 10% by weight, preferably 0.1 to 5%, and more preferably 0.1 to 3% of a homopolymer obtainable by polymerising a monomer A or B or a copolymer obtainable by copolymerising monomers A and B, where A represents:
a) a dialkylaminoalkyl acrylate of formula I 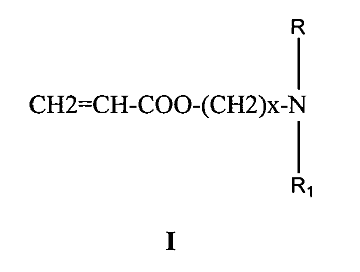 where R and R1, which may be the same or different, represent a straight-chain or branched alkyl group C1-4
   and x is an integer between 2 and 10 or a salt thereof with hydrochloric, nitric, sulphuric or phosphoric acid, or an organic acid;
   or:
b) its quaternary ammonium salt of formula II 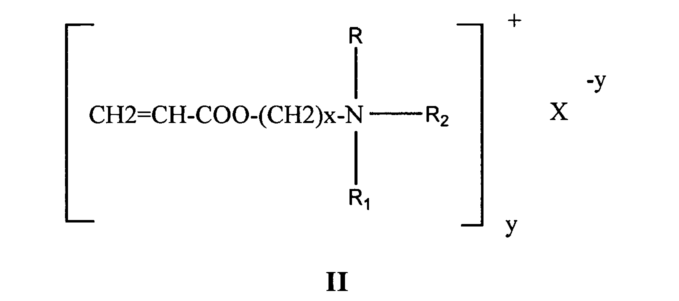
wherein R and R1 are as defined above and R2 is a straight-chain or branched alkyl group C1-4, y takes values from 1 to 3 and X is Cl⁻, NO₃⁻, SO₄²⁻, PO₄³⁻, CH₃-OSO₃⁻ or C₂H₅-O-SO3⁻
B represents:
a) a dialkylaminoalkyl methacrylate of formula III 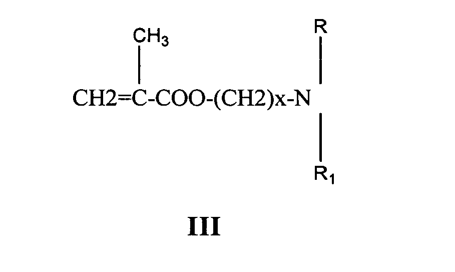 wherein R and R1 are as defined above, and x is an integer between 2 and 10 or a salt of compound III with hydrochloric, nitric, sulphuric or phosphoric acid, or an organic acid;
   or:
b) its quaternary ammonium salt of formula IV 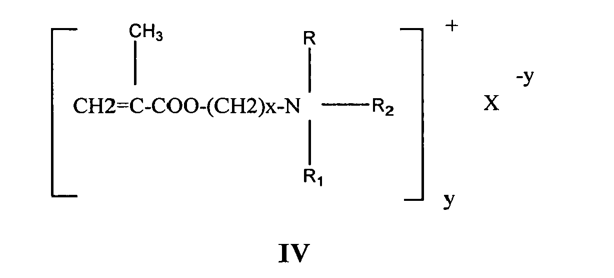
wherein R, R1, R2 and y are as defined above, and X may be Cl⁻, NO₃⁻, SO₄²⁻, PO₄^{3-,} CH₃-OSO₃⁻, C₂H₅-O-SO3.

The polymer according to the invention may also contain a crosslinking agent, such as a compound containing two or more unsaturations. The choice will preferably fall on methylenebisacrylamide, diallyldialkylammonium chloride, polyalkenyl polyethers of polyalcohols, or allyl acrylates. The preferred crosslinking agent is methylenebisacrylamide.

A preferred polymer according to the invention is a polymer obtained from a monomer B which is preferably a dialkylaminoalkyl methacrylate or a quaternary ammonium derivative thereof.

The method according to the invention is used to prepare o/w emulsions, wherein both the water phase and the oil phase can contain well-known ingredients. Said emulsions can have a very variable viscosity, ranging from a few hundred to a few thousand centipoise, measured with a Brookfield viscosimeter at 20 rpm.

The pH of the compositions can range from very low values, as in the case of emulsions containing alpha-hydroxyacids (pH 2-3.5), to fairly low values, as in the case of tanning emulsions containing dihydroxyacetone (pH 3-4.5), or only slightly acid, as in the case of sunscreen emulsions or simple skin care emulsions (pH 5-7).

The fatty phase of the emulsion may contain the following ingredients:
i) hydrocarbons such as paraffin, mineral oils and analogues;
ii) oils, butters and natural waxes such as avocado oil, sunflower seed oil, almond oil, apricot seed oil, karité butter, evening primrose oil, blackcurrant oil, borage oil, jojoba oil, safflower oil, wheatgerm oil, macadamia oil, rice husk oil, sesame seed oil, castor oil, coconut oil, unsaponifiable fractions of olive, avocado and soya, cocoa butter, beeswax, candelilla wax, carnauba wax and analogues;
iii) silicone oils such as dimethicones, cyclomethicones, dimethiconols, alkyldimethicones, and analogues;
iv) saturated or unsaturated, straight-chain or branched esters of aliphatic acids, or of aromatic or alkylaromatic acids, having 1 to 25 carbon atoms, with mono- or polyhydroxylated, saturated or unsaturated, straight-chain or branched aliphatic alcohols, having 1 to 25 carbon atoms, such as octyldodecyl-neopentanoate, pentaerythritol-dioleate, trimethylolpropane trioleate, triisostearyl citrate, diacetin, triacetin, 2-ethylhexyl-acetate, neopentylglycol-oleate, triethylene glycol diacetate, isopropyl myristate, isopropyl palmitate, bis-diglycerylcaprylate/caprate/isostearate/stearate/ hydroxystearate, bis-diglyceryl adipate, dioctyl maleate, di-(2-ethylhexyl)-malate, (C12-1)alkyl benzoates, cetyl stearyl octanoate, cetylstearyl isononanoate, 2-ethylhexyl-palmitate, 2-ethylhexyl-stearate, C8-10 triglyceride, PEG7-glyceryl cocoate, and analogues;
v) amides such as those mentioned in EP 0 748 623, especially N,N-diethyl-3-methylbenzamide and ethyl 1-[(N-acetyl-N-butyl)amino]-propionate;
vi) alcohols containing 6 to 35 carbon atoms, such as cetyl alcohol, stearyl alcohol, behenyl alcohol, octyldodecyl alcohol, 3,5,5-trimethylhexyl alcohol, 2-butoxyethanol, 2-phenoxyethanol, 2-ethyl-1,3-hexanediol, and analogues;
vii) ethers of fatty alcohols containing 8 to 40 carbon atoms, such as di-n-octylether;
viii) glycol butylethers such as propylene glycol-tert-butylether, diethylene glycol butylether, a (polypropylene glycol)3-53 butylether, and analogues;
ix) esters of (C1-6)alkylethers such as diethylene glycol butylether acetate, propylene glycol methylether acetate, and analogues.

For the purposes of this invention, the substances from i) to ix), all of which are easily available on the market, can be used individually, or as one of the possible mixtures thereof, such as the wax mixtures known under the trade name CUTINA^{™} (Henkel).

The oily ingredient is generally used in quantities of between approx. 0.5 and 99.5% or more of the total weight of the composition.

A preferred group of oily ingredients are esters of saturated or unsaturated, straight-chain or branched aliphatic acids, or of aromatic or alkylaromatic acids, having 1 to 25 carbon atoms, which said acids can optionally be hydroxylated and/or ethoxylated with mono- or polyhydroxylated saturated or unsaturated aliphatic alcohols having 1 to 25 carbon atoms.

Another preferred group of oily ingredients are amide derivatives, including N,N-diethyl-3-methylbenzamide and ethyl 1-[(N-acetyl-N-butyl)amino]-propionate.

A third preferred group of oily ingredients are mixtures of esters of saturated or unsaturated straight-chain or branched aliphatic acids, or of aromatic or alkylaromatic acids, having 1 to 25 carbon atoms, with mono- or polyhydroxylated, saturated or unsaturated, straight-chain or branched aliphatic alcohols having 1 to 25 carbon atoms, and N,N-diethylmethylbenzamides and/or ethyl 1-(N-acetyl-N-butyl)-propionate.

A fourth preferred group of oily ingredients consists of silicone oils. The quantity of the oily phase can range between 5 and 40%.

The preparation of the emulsions claimed includes the addition of one or more conventional emulsifiers, available on the market. They may be cationic or non-ionic emulsifiers. The latter are, for example, ethoxylated compounds of derivatives of natural oils, such as castor oil (7)OE hydrogenate (ARLACEL^{™} 989, ICI); mono- and diglycerides of ethoxylated and non-ethoxylated fatty acids, such as glyceryl stearate (CUTINA^{™} GMS, Henkel) and glyceryl(20)OE stearate (CUTINA^{™} E-24, Henkel); ethoxylated sorbitan esters TWEEN^{™}, ICI and CRILLET^{™}, Croda) and non-ethoxylated sorbitan esters (SPAN^{™}, ICI and CRILL^{™}, Croda); esters of polyglycerol with fatty acids, such as triglyceryl diisostearate (LAMEFORM^{™} TGI, Henkel) and triglyceryl distearate (CITHROL^{™} 2623, Croda); esters of glucose, methylglucose and saccharose with ethoxylated and non-ethoxylated fatty acids, such as methylglucose dioleate (GLUCATE^{™} DO, Amerchol) and methylglucose (20)OE sesquistearate (GLUCAMATE^{™} SS E-20, Amerchol); ethers of glucose and its oligomers, optionally esterified with aliphatic acids C10-30, such as triglyceryl methylglucose distearate (TegoCare™ 450, Goldschmidt), or etherified with aliphatic alcohols C8-30, such as cetylstearyl glucoside (MONTANOV^{™} 68, Seppic); ethoxylated fatty acids (MYRJ^{™}, ICI); ethoxylated fatty alcohols (BRIJ^{™}, ICI); lanolin and its ethoxylated and non-ethoxylated derivatives, such as lanolin (30)OE (AQUALOSE^{™} L 30, Westbrook); alkylglycol/polyethylene glycol copolymers, such as copolymer PEG-45/dodecylglycol (ELFACOS^{™} ST 9, Akzo); silicone emulsifiers (Silicone Fluid 3225 C, Dow Corning; ABIL^{™} WS05, Th. Goldschmidt AG); fluoride emulsifiers (FOMBLIN^{™} Ausimont). Cationic emulsifiers which can be advantageously used in the compositions are quaternary ammonium salts having one or two fatty alkyl chains with a number of carbon atoms ranging between 12 and 22, preferably between 14 and 22, and a counter-ion selected from chloride, bromide, iodide, acetate, phosphate, nitrate, sulphate, methylsulphate, ethylsulphate, tosylate, lactate, citrate, and glycolate. Quaternary ammonium derivatives with 2 alkyl chains are preferred, due to their low irritant power. Examples of preferred cationic emulsifiers are distearyl dimethyl ammonium chloride, dimyristyl dimethyl ammonium chloride and dipalmityl dimethyl ammonium chloride, present on the market as Genamin^{™}, Clariant and Varisoft^{™}, Degussa.

The quantity of emulsifiers which can be used ranges between approx. 0.1 and approx. 20% of the total weight of the composition, preferably between 0.1 and 10% and more preferably between 0.1 and 6%. They can be used individually or mixed together.

The fatty phase of the cosmetic compositions according to the invention can also include, in combination, one or more anti UV-A or anti UV-B sunscreens chosen, for example, from derivatives of benzylidene camphor, derivatives of dibenzoylmethane, esters and salts of alkoxycinnamic acids, benzophenone derivatives, diphenylcyanoacrylates, derivatives of salicylic acid, derivatives of benzimidazole sulphonic acid, derivatives of p-aminobenzoic acid, 2-(2H-benzotriazol-2-yl)-4-methyl-6-{2-methyl-3-[1,3,3,3-tetramethyl-1-[(trimethylsilyl)oxy]-disiloxanyl]-propyl}-phenol (silatriazole), and oxides of metals with an atomic number between 21 and 30.

Representative examples of the sunscreens belonging to the classes of compounds mentioned above are benzylidene camphor derivatives such as bicyclo[2.2.1]heptan-2-one 1,7,7-trimethyl-3-[(4-methylphenyl)methylene]; 3-(4'-trimethylammonium)benzylidene-bornan-2-one methylsulphate; and 3,3'-(1,4-phenylendimethin)-bis-(7,7-dimethyl-2-oxobicyclo[2,2,1]heptan-1-methanesulphonic) acid, commercially known as EUSOLEX^{™} 6300, MEXORIL^{™} SK and MEXORIL^{™} SX respectively; 4-methoxy-4'-tert-butyl-dibenzoylmethane, a derivative of dibenzoylmethane commercially known as PARSOL^{™} 1789; 2-ethylhexyl-4-methoxycinnamate and 4-methoxycinnamic acid diethanolamine salt, alkoxycinnamic acid derivatives commercially known as PARSOL^{™} MCX and BERNEL^{™} HYDRO; 2,4-dihydroxybenzophenone, 2-hydroxy-4-methoxy-benzophenone and 5-benzoyl-4-hydroxy-2-methoxy-benzene sulphonic acid, benzophenone derivatives commercially known as UVASORB^{™} 20H, UVASORB^{™} MET; 2-ethylhexyl 2-cyano-3,3-diphenylacrylate, commercially known as UVINUL^{™} N-539 or EUSOLEX OCR; 2-ethylhexyl 2-hydroxybenzoate, (+)3,3,5-trimethylcyclohexyl salicylate and salicylic acid triethanolamine salt, salicylic acid derivatives commercially known as ESCALOL^{™} 587, KEMESTER^{™} HMS and SUNAROME^{™} W; ethyl 2-ethylhexyl 4-dimethylamino-benzoate, N,N-bis-(2-hydroxypropyl)-benzoate and 4-aminobenzoic acid PEG 25, p-aminobenzoic acid derivatives commercially known as UVASORB^{™} DMO, AMERSCREEN^{™} P and UVINUL^{™} P25; triazine derivatives sold as UVASORB^{™} HEB by from 3V Sigma, TINOSORB^{™} S by CIBA and UVINUL^{™} T150 by BASF; benzalmalonate derivatives sold as Parsol SLX by Hoffmann-LaRoche.

Among the oxides of metals having an atomic number between 21 and 30, titanium dioxide (TiO₂) and zinc oxide (ZnO) are preferred.

Said oxides are preferably used in micronised form, with a particle size not exceeding approx. 100 nm for TiO₂, and between approx. 15 and approx. 300 nm for ZnO. Even more preferably, the particle size of TiO₂ is between approx. 5 and approx. 50 nm. Titanium dioxide may have an anatase, rutyl or amorphous structure. These micronised metal oxides can be used as such or coated with other agents such as Al₂O₃ or aluminium salts with aliphatic fatty acids C10-18 or silicones.

These products are readily available on the market. For example, TiO₂ is micronised and sold under the trade name P25 (Degussa), while TiO₂ is coated with aluminium stearate and sold as METRES100T (Taika Corp.), while the version coated with Al₂O₃ is known as UFTR (Miyoshi). Micronised ZnO is obtainable as Z-COTE^{™} (sunSMART) or SPECTRAVEIL^{™} (Tioxide).

The emulsions claimed can contain 40 to 95% water, preferably 60 to 90%, and even more preferably 70 to 90% by weight.

The compositions according to the invention comprise 0.1 to 10%, preferably 0.1 to 5%, and more preferably 0.1 to 3% of a polymer or copolymer as defined above.

The class of cationic polymers of trimethylaminoethyl methacrylate cross-linked with methylenebisacrylamide is preferred. A commercial example of a cross-linked cationic polymer belonging to this class is Synthalen CR (3V Sigma S.p.A.), which corresponds to the name CTFA Polyquaternium-37.

Dihydroxyacetone (DHA) can be added to the water phase, possibly in the presence of erythrulose, to give a tan with a less yellowish hue than the shade obtained with DHA alone. Said artificial tanning products can be present in quantities of between approx. 0.1 and approx. 20% by weight of the composition, preferably between 2 and 10%, and more preferably between 2 and 7%. In order to be stable, compositions containing DHA must have a pH of less than 4.5, and preferably less than 4.0. At these pH values, it is not easy to achieve good viscosity and stability values using anionic rheology modifiers.

One or more alphahydroxyacids (AHA), more specifically identified as 2-hydroxycarboxylic acids with the following formula, can be added to the formulations according to the invention:

(R₁)(R₂)C(OH)COOH

wherein R1 and R2 may be the same or different and are selected from H, F, Cl, Br, alkyl and arylalkyl, wherein alkyl and arylalkyl may have 1 to 29 carbon atoms and may be straight-chain, branched or cyclic, and may contain groups such as OH and COOH. Said organic acids may be present in quantities of between approx. 0.1 and approx. 20% by weight of the composition, preferably 2 to 15% and more preferably 2 to 10%. In order to present good exfoliating activity, compositions containing AHA must have a pH of between 3.5 and 2. At these pH values, it is practically impossible to achieve good viscosity and stability values using anionic rheology modifiers.

The cosmetic compositions according to the invention can also include other conventional ingredients such as humectants, like glycerin, diglycerin, propylene glycol or 1,3-butylene glycol, in quantities ranging from approx. 0.1 to approx. 30% by weight of the composition; sequestering agents, such as EDTA salts, in quantities not exceeding 1% by weight of the composition; antioxidants, such as tocopherols and esters thereof, hydroxytoluene butylate or butylhydroxyanisol, in quantities not exceeding 2% by weight of the composition; insect-repellent agents such as diethyltoluamide in quantities of between 2 and 20%; moisturising agents in quantities not exceeding 5% by weight of the composition; agents to adjust the pH to the required values, such as sodium or potassium citrate, sodium or potassium hydroxide, or citric acid monohydrate, in quantities not exceeding 1% by weight of the composition; preservatives, such as 2-bromo-2-nitro-propanediol, sodium dehydroacetate, isothiazolone, imidazolidinyl urea, diazolidinylurea, parabens and hydantoin derivatives (GLYDANT^{™} Lonza), sorbic acid, benzoic acid and their salts, chlorhexidine and its salts, phenoxyethanol, benzyl alcohol, and analogues, in quantities not exceeding 10% by weight of the composition. Perfumes and colorants can also be added.

The cosmetic compositions according to the invention can also be prepared according to known methods. In particular, as these are O/W emulsions, it is preferable to prepare the two phases separately, dissolving or dispersing the required lipophilic or hydrophilic constituents/ingredients in each of them, and then mixing them. The cationic polymer can be dispersed in the water phase, before the homogenisation stage, or in the emulsion already formed.

Examples of these compositions are sunscreens, tanning creams, wrinkle creams, exfoliant creams, day creams, moisturising creams, insect-repellent emulsions, lipstick, mascara and analogues.

They contain the ingredients in the weight ratios stated above, and any other ingredients compatible with them which are conventionally used in the said cosmetic preparations.

### Evaluation of the Water resistance

100 mg of emulsion containing an UV filter is applied to a microscope slide with an area of 50 cm² and covered with Transpore^{™} Surgical Tape 1527 manufactured by 3M. After 20 minutes in the dark, the slide is immersed in tap water at 25 ± 1°C, and the water is allowed to recirculate to simulate moderate activity. After 20 minutes' immersion the slide is left to dry in the air, protected from the light. The complete immersion/drying cycle for each slide is repeated up to 4 times in total. For each emulsion, a control slide (blank) is prepared and stored in the dark throughout the duration of the experiment.

At the end of the immersion/drying cycles the emulsion left on the slides is recovered, washed with cotton, wet with absolute ethanol and then left fully immersed for two hours in the ethanol solution obtained, protected against the light. The final solutions are then analysed for UV filter content by UV/Visible spectroscopy. The water resistance of the emulsions, calculated in terms of retention of the UV filter, is obtained from the ratio between the mean absorbance value of four samples immersed (for each emulsion) and the absorbance of the blank (sample not immersed in water).

### Measurement of viscosity

The viscosity of the emulsions prepared was evaluated with a Brookfield viscosimeter using the appropriate impeller, according to the value to be measured, on samples left to stand overnight, after preparation, and thermostated at 25°C. The measurement was performed at 20 rpm, and the value expressed in cps.

### EXAMPLES

Some representative examples of cosmetic compositions containing the associations according to the invention are given below, together with the water resistance values measured *in vitro.* The quantities of the individual ingredients are expressed as percentages of the total weight of the composition. The individual ingredients are indicated by their CTFA name.

### EXAMPLES 1-6

| **Sunscreen emulsions** | **Example 1** | **Example 2** | **Example 3** | **Example 4** | **Example 5** | **Example 6** |
|---|---|---|---|---|---|---|
| Distearyldimonium Chloride | | | | | | 3 |
| Glyceryl Monostearate | 0,5 | 0,5 | 0,5 | 0,5 | 0,5 | 1 |
| Steareth-100 | 0,4 | 0,4 | 0,4 | 0,4 | 0,4 | |
| Dimethicone | 1 | 1 | 1 | 1 | 1 | |
| Cetyl Alcohol | 3 | 3 | 3 | 3 | 3 | 1 |
| C₁₂₋₁₅ Alkyl Benzoate | 2 | 2 | 2 | 2 | 2 | 4 |
| Ethylhexyl Palmitate | | | | | | 4 |
| Isononyl Isononanoate | | | | | | |
| Propylene Glycol Dicaprylate/Dicaprate | | | | | | 2 |
| Butyrospermum Parkii | | | | | | |
| BHT | 0,1 | 0,1 | 0,1 | 0,1 | 0,1 | 0,1 |
| PVP/Eicosene Copolymer | | | | | 1 | |
| Ethylhexyl Methoxycinnamate | | | 3 | 3 | | |
| Diethylhexyl Butamido Triazone | 3 | 3 | | | 3 | 3 |
| H₂O | Balance | Balance | Balance | Balance | Balance | Balance |
| Polyquaternium-37 (SYNTHALEN CR) | | 0,3 | | 0,3 | | 0,3 |
| Carbomer 940 | 0,2 | | 0,2 | | 0,2 | |
| Glycerin | 3 | 3 | 3 | 3 | 3 | 3 |
| Allantoin | 0,2 | 0,2 | 0,2 | 0,2 | 0,2 | 0,2 |
| Lactic Acid (40%) | | to pH 5,5-6,0 | | to pH 5,5-6,0 | | |
| AMP | to pH 5.5-6.0 | | to pH 5.5-6.0 | | to pH 5.5-6.0 | to pH 5.5-6.0 |
| Phenoxyethanol & Parabens | 1 | 1 | 1 | 1 | 1 | 1 |
| **Brookfield viscosity, 20 rpm, 25°C (cps)** | **24500** | **9200** | **20500** | **12000** | **30000** | **26000** |
| **UV filter recovery after 40 sec. immersion (%)** | **53** | **96** | **50** | **97** | **96** | **97** |
| **UV filter recovery after 80 sec. immersion (%)** | **33** | **94** | **32** | **90** | **89** | **94** |

Examples 1 and 3 both represent a classic anionic emulsion viscosified with an anionic polymer. Their water resistance does not comply with COLIPA guidelines. To obtain water resistance in line with the COLIPA guidelines it is necessary to add 1% of PVP/eicosene copolymer, thus modifying the biodegradability of the product (1.2% of polymer as against 0.3% of the emulsions with Synthalen CR) and, above all, the "skin feeling" of the final cream, which is sticky both at the spreading stage and after application. All three examples with Synthalen CR (2, 4 and 6) demonstrated excellent water resistance, together with excellent sensory properties during spreading and after application.

### EXAMPLES 7-12

| **Tanning emulsions with sunscreen** | **Example 7** | **Example 8** | **Example 9** | **Example 10** | **Example 11** | **Example 12** |
|---|---|---|---|---|---|---|
| Polyglyceryl-10 Pentastearate & Behenyl | | | | | | |
| Alcohol & Stearamidopropyl Dimethylamine | | | | | | |
| Lactate | 2,5 | | | | | |
| Distearyldimonium Chloride | | | | 3 | | 3 |
| Glyceryl Monostearate | | | | 1 | | 1 |
| Cetearyl Alcohol & Ceteareth-20 | | | 3 | | 3 | |
| Paraffinum Liquidum | | | 6 | | 6 | |
| Ethylhexyl Sterate | | | 2 | | 2 | |
| Ethylhexyl Palmitate | | | | 4 | | 4 |
| Ethylhexyl Cocoate | 5 | | | | | |
| Cetearyl Alcohol | | | 2 | 1 | 2 | 1 |
| Neopentyl Glycol Dicaprylate/Dicaprate | 5 | | | | | |
| Dimethicone | 1 | | | | | |
| Ceteareth-20 | | 0,5 | | | | |
| C₁₂₋₁₅ Alkyl Benzoate | | 5 | 5 | 4 | 5 | 4 |
| Glyceryl Stearate | | 1 | | | | |
| Propylene Glycol Dicaprylate/Dicaprate | | 5 | | 4 | | 4 |
| Diethylhexyl Butamido Triazone | 3 | 3 | 3 | 3 | | |
| Ethylhexyl Triazone | | | | | 3 | 3 |
| Stearyl dimethicone | | 2 | | | | |
| H2O | Balance | Balance | Balance | Balance | Balance | Balance |
| Polyquaternium-37 (Synthalen CR) | 0,2 | 0,3 | 0,2 | 0,3 | 0,2 | 0,3 |
| Butylene Glycol | 3 | | | | | |
| Dihydroxyacetone | 3 | 3 | 3 | 3 | 3 | 3 |
| Glycerin | | 5 | 5 | 5 | 5 | 5 |
| Allantoin | 0,2 | 0,2 | 0,2 | 0,2 | 0,2 | 0,2 |
| Lactic Acid | to pH 3,5 | to pH 3,5 | to pH 3,5 | to pH 3,5 | to pH 3,5 | to pH 3,5 |
| Sodium Sulfite | 0,2 | 0,2 | 0,2 | 0,2 | 0,2 | 0,2 |
| Cyclopentasiloxane | | 5 | | | | |
| Phenoxyethanol & Parabens | 1 | 1 | 1 | 1 | 1 | 11 |
| **Brookfield viscosity, 20 rpm, 25°C (cps)** | **9400** | **8000** | **25000** | **11000** | **23000** | **10500** |
| **UV Filter recovery after 40 sec. immersion (%)** | **97** | **96** | **94** | **95** | **93** | **95** |
| **UV Filter recovery after 80 sec. immersion (%)** | **90** | **88** | **88** | **86** | **87** | **85** |

### EXAMPLES 13-18

| **Exfoliant face creams with sunscreen** | **Example 13** | **Example 14** | **Example 15** | **Example 16** | **Example 17** | **Example 18** |
|---|---|---|---|---|---|---|
| Distearyldimonium Chloride | 3 | | 3 | 3 | | |
| Glyceryl Monostearate | 1 | 0,5 | 1 | 1 | 0,5 | 0,5 |
| Steareth-100 | | 0,4 | | | 0,4 | 0,4 |
| Stearyl Alcohol | | 1,5 | | | 1,5 | 1,5 |
| Cetyl Alcohol | 1 | 1,5 | 1 | 1 | 1,5 | 1,5 |
| C12-15 Alkyl Benzoate | 4 | 2 | 4 | 4 | 2 | 2 |
| Ethylhexyl Palmitate | 4 | | 4 | 4 | | |
| Propylene Glycol Dicaprylate/Dicaprate | 2 | | 2 | 2 | | |
| Diethylhexyl Butamido Triazone | 3 | 3 | 3 | 3 | 3 | |
| Ethylhexyl Triazone | | | | | | 3 |
| Dimethicone | | 1 | | | 1 | 1 |
| H2O | Balance | Balance | Balance | Balance | Balance | Balance |
| Polyquaternium-37 (Synthalen CR) | 0,5 | 0,5 | 0,75 | 0,75 | 0,5 | 0,5 |
| Propylene Glycol | | 3 | | | 3 | 3 |
| Glycerin | 3 | | 3 | 3 | | |
| Allantoin | 0,2 | 0,2 | 0,2 | 0,2 | 0,2 | 0,2 |
| Aqua & Possiflora Quadrangularis & Citrus | | | | | | |
| Medica Limonum | 0,5 | 0,5 | 0,75 | 0,75 | 0,75 | 0,75 |
| Glycolic Acid | 2 | 2 | 5 | 10 | 10 | 10 |
| NaOH | to pH 3,5 | to pH 3,5 | to pH 3,5 | to pH 3,5 | to pH 3,5 | to pH 3,5 |
| Cyclopentasiloxane | 3 | 3 | 3 | 3 | 3 | 3 |
| Diazolidinyl Urea | 0,2 | 0,2 | 0,2 | 0,2 | 0,2 | 0,2 |
| Phenoxyethanol & Parabens | 0,4 | 0,4 | 0,4 | 0,4 | 0,4 | 0,4 |
| **Brookfield Viscosity** | **9500** | **10000** | **12500** | **11500** | **12500** | **11800** |
| **UV Filter recovery after 40 sec. immersion (%)** | **97** | **95** | **96** | **93** | **91** | **90** |
| **UV Filter recovery after 80 sec. immersion (%)** | **88** | **86** | **87** | **84** | **82** | **83** |

## Claims

1. Method for giving oil-in-water emulsions, designed to be applied to the skin and/or hair, a high degree of water resistance, consisting of incorporating in the emulsions 0.1% to 10% of a polymer obtainable by homopolymerisation of a monomer A or B or copolymerisation of A with B, wherein A represents:
a) a dialkylaminoalkyl acrylate of formula I 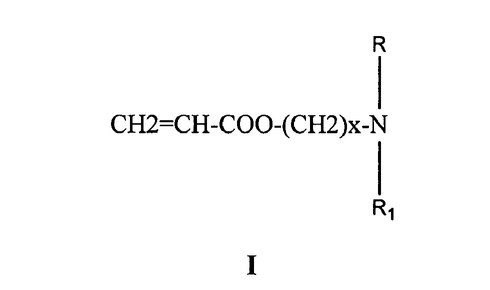 where R and R1, which may be the same or different, represent a straight-chain or branched alkyl group C1-4
and x is an integer between 2 and 10 or a salt thereof with hydrochloric, nitric, sulphuric or phosphoric acid, or an organic acid;
or:
b) its quaternary ammonium salt of formula II 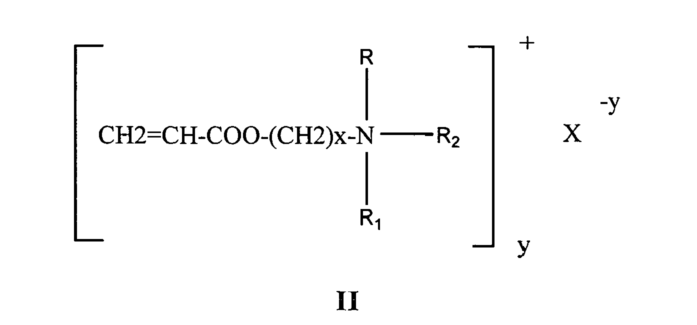
wherein R and R1 are as defined above and R2 is a straight-chain or branched alkyl group C1-4, y takes values from 1 to 3 and X is Cl⁻, NO₃⁻, SO₄²⁻, PO₄³⁻, CH₃-OSO₃⁻ or C₂H₅-O-SO3⁻
B represents:
a) a dialkylaminoalkyl methacrylate of formula III 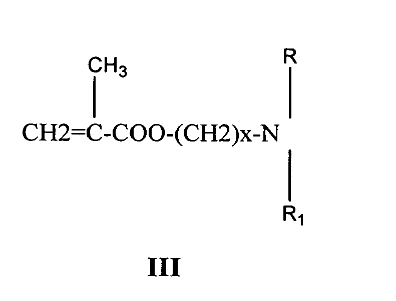 wherein R and R1 are as defined above, x is an integer between 2 and 10 or a salt of compound III with hydrochloric, nitric, sulphuric or phosphoric acid, or an organic acid;
or:
b) its quaternary ammonium salt of formula IV 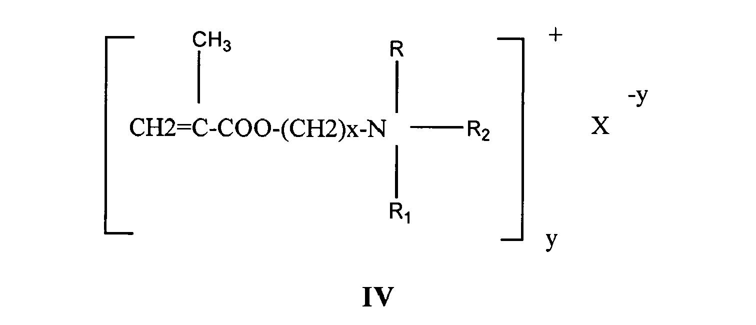
wherein R, R1, R2 and y are as defined above, and X can be Cl⁻, NO₃⁻, SO₄²⁻, PO₄³⁻, CH₃-OSO₃⁻, C₂H₅-O-SO3.

2. Method as claimed in claim 1, wherein the polymer or copolymer is crosslinked by means of a crosslinking agent.

3. Method as claimed in claim 1 or 2, wherein the crosslinking agent is selected from methylenebisacrylamide, diallyldialkylammonium chloride, polyalkenyl polyethers of polyalcohols, and allyl acrylates.

4. Method as claimed in claim 3, wherein the crosslinking agent is methylenebisacrylamide.

5. Method as claimed in any of claims 1 to 3, wherein the polymer is a homopolymer of trimethylammonium ethyl methacrylate chloride cross-linked with methylenebisacrylamide.

6. Cosmetic compositions obtainable by the method described in claims 1-5.

7. Compositions as claimed in claim 6, in the form of oil-in-water emulsions.
